(19) Europäisches Patentamt
European Patent Office
Office européen des brevets

(11) Veröffentlichungsnummer: **0 267 467**
**B1**

(12) # EUROPÄISCHE PATENTSCHRIFT

(45) Veröffentlichungstag der Patentschrift: **18.10.89**

(51) Int. Cl.⁴: **C07D 333/68**, C07C 153/063, C09B 29/033

(21) Anmeldenummer: **87115393.8**

(22) Anmeldetag: **21.10.87**

(54) **Verfahren zur Herstellung von Thiophenverbindungen.**

(30) Priorität: **03.11.86 DE 3637223**

(43) Veröffentlichungstag der Anmeldung:
**18.05.88 Patentblatt 88/20**

(45) Bekanntmachung des Hinweises auf die Patenterteilung:
**18.10.89 Patentblatt 89/42**

(84) Benannte Vertragsstaaten:
**CH DE FR GB LI**

(56) Entgegenhaltungen:
**EP-A- 0 166 566**
**DE-A- 2 333 447**

**CHEMISCHE BERICHTE, Band 101, Nr. 6, 1968, Seiten 1933-1939, Verlag Chemie GmbH, Weinheim, DE; K. GEWALD et al.: "2-Amino-thionaphthene"**

(73) Patentinhaber: **BAYER AG, D-5090 Leverkusen 1 Bayerwerk(DE)**

(72) Erfinder: **Hamprecht, Rainer, Dr., Im Kerberich 25, D-5068 Odenthal(DE)**

**EP 0 267 467 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein Verfahren zur Herstellung von 2-Aminothiophenen der Formel

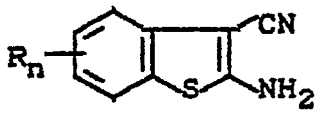

(I)

worin
R einen Substituenten und
n eine Zahl von 0 bis 4 bedeuten.

Es ist bereits bekannt, Verbindungen dieses Typs durch die sogenannte "Gewald-Reaktion" und anschließende Aromatisierung herzustellen (vgl. Chem. Ber. 98, 3571 (1965) und 101, 1933 (1968)).

Das Prinzip dieser Synthese sei an Hand des folgenden Reaktionsschemas beispielhaft erläutert:

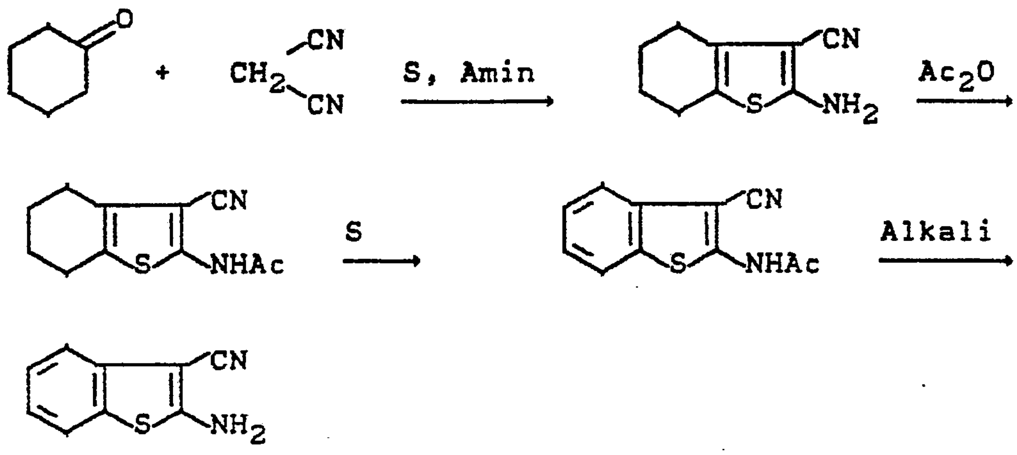


Diese orginelle Methode weist jedoch den Nachteil auf, daß sie über viele Stufen verläuft, von denen insbesondere die erste und vorletzte nur verhältnismäßig geringe Ausbeuten liefern. Die Gesamtausbeute (bezogen auf Cyclohexanon) beträgt deshalb auch nur knapp 10%.

Es wurde nun gefunden, daß man 2-Aminothiophene der Formel (I) wesentlich vorteilhafter erhält, wenn man Thioamide der Formel

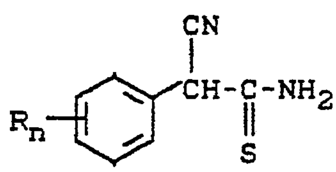

(II)

mit Dehydrierungsmitteln ringschließend umsetzt.

Geeignete Substituenten R in den Formeln (I) bzw. (II) sind insbesondere nichtionische Typen, wie sie vor allem in der Farbstoffchemie üblich sind.

Beispielhaft seien erwähnt: F, Cl, Br, I, $C_1$–$C_4$-Alkyl, $NO_2$, CN, $SO_2$–$C_1$–$C_4$-Alkyl und $CF_3$.

Darüber hinaus können zwei benachbarte Reste R gemeinsam einen ankondensierten carbo- oder heterocyclischen Ring bilden.

Bevorzugte Verfahrensprodukt entsprechen der Formel

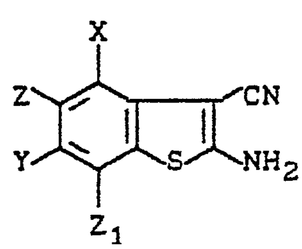

(Ia)

worin

X H, Cl, Br, $CF_3$ oder $SO_2$–$C_1$–$C_2$-Alkyl,
Y H, Cl, Br oder $NO_2$ und
Z/$Z_1$ Cl, Br und insbesondere H bedeuten.

Als Dehydrierungsmittel zur Durchführung der erfindungsgemäßen Cyclisierung kommen insbesondere freies Halogen ($J_2$, $Br_2$, $Cl_2$) und halogenabspaltende Verbindungen (z.B. N-Bromsuccinimid) in betracht.

Bevorzugt sind Iod und vor allem Brom.

Im allgemeinen werden äquimolare Mengen davon eingesetzt.

Die Reaktion kann in einem breiten Temperaturbereich (etwa –20 bis; 100°C) durchgeführt werden. Vorzugsweise arbeitet man bei Raumtemperatur, d.h. also bei etwa 10 bis 30°C.

Zweckmäßigerweise wird die Umsetzung in einem geeigneten unter Reaktionsbedingungen indifferenten Lösungsmittel durchgeführt. Bevorzugt sind organische Lösungsmittel , wie z.B. Methanol, Ethanol, Isopropanol, $CH_2Cl_2$, $C_2H_2Cl_2$, $CHCl_3$, $CCl_4$, Toluol, Chlorbenzol.

Besonders bevorzugt sind unpolare Typen

Im übrigen empfiehlt es sich, die Ringschlußreaktion in Gegenwart eines Säureakzeptors, wie z.B. organischen und anorganischen Basen, vorzunehmen.

Geeignete Basen sind: Trialkylamine, Pyridin sowie Alkali- und Erdalkalioxide, -hydroxide, -carbonate und vorzugsweise -hydrogencarbonate, wie z.B. $NaHCO_3$.

Bei der praktischen Durchführung des Verfahrens geht man beispielsweise so vor, daß man in eine Lösung bzw. Dispersion des Thioamids und des Natriumhydrogencarbonats in Methylenchlorid bei Raumtemperatur eine Lösung von Brom in Methylenchlorid zutropft und solange nachrührt, bis die Reaktion beendet ist.

Es ist durchaus als überraschend anzusehen, daß dieser Ringschluß so glatt verläuft, da aufgrund ähnlicher bekannter Reaktionen (vgl. J. Org. Chem. 31, 2654 (1966)) erwartet werden konnte, daß zumindest zum Teil Dimerisierung des Ausgangsmaterials zu Verbindungen der folgenden Formel eintritt:

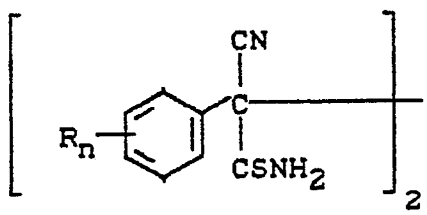

Die Thioamide der Formel (II) sind bislang nicht in der Literatur beschrieben worden. Es wurde nun im Rahmen eines weiteren Gegenstandes dieser Erfindung gefunden, daß man diese neuen Verbindungen leicht dadurch erhält, daß man Verbindungen der Formel

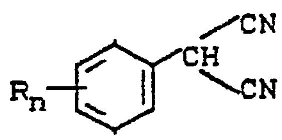 (III)

mit Verbindungen der Formel

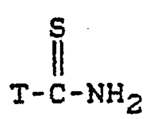 (IV)

worin
T $NH_2$, Aryl oder Alkyl, vorzugsweise Methyl, bedeutet,
in Gegenwart einer Säure umsetzt.

Auch diese Umsetzung erfolgt vorteilhafterweise in einem Lösungsmittel. In diesem Falle sind jedoch besonders dipolare aprotische Lösungsmittel, wie z.B. DMF geeignet.

Geeignete Säuren sind vor allem Halogenwasserstoffsäuren, wie z.B. Chlorwasserstoff.

Die Verbindungen der Formel (IV), vorzugsweise Thioacetamid, werden in äquimolaren Mengen sowie bis zu einem Überschuß von 100% eingesetzt.

Es ist überraschend, daß bei der Umsetzung von (III) mit (IV) – entgegen anderer Befunde an ähnlichen Systemen (vgl. J.A.C.S. 82, 2656 (1960)) – auch bei einem Überschuß von (IV) nur das Monothioamid entsteht.

Durch diesen nicht vorhersehbar problemlosen Reaktionsverlauf ergeben sich in Abhängigkeit von der Art des eingesetzten Ausgangsmaterials Gesamtausbeuten an dem Verfahrensprodukt der Formel

(I) über alle Stufen hinweg (d.h. 1. Herstellung von (III), 2. Umsetzung von (III) mit (IV), 3. Cyclisierung von (II)) von 40 bis 60%.

Das bedeutet eine wesentliche Ausbeuteverbesserung gegenüber dem bekannten "Gewald-Verfahren", das – wie oben gezeigt – nur Ausbeuten von weniger als 10% liefert - und auch das nur am unsubstituierten Typ mit $R_n$ = H.

Die Herstellung der als Ausgangsmaterial benötigten Verbindungen der Formel (III) erfolgt nach an sich bekannten Methoden (vgl. DE-A 2 854 197 = GB-A 2 037 286; J.A.C.S. 72 1853 (1950), und 74, 3443 (1952) sowie W. Hindermayr, Dissertation, Ludwig-Maximilian-Universität, München 1967) und wird in den nachfolgenden Beispielen näher erläutert.

Die Verfahrensprodukte der Formel (I) liefern Diazokomponenten, die sich nach üblichen Methoden in teilweise bekannte Azofarbstoffe überführen lassen (vgl. DE-A 2 333 447 = GB-A 1 434 654).

Beispiel 1

a) Darstellung von 2-Amino-3-cyan-6-nitrobenzo-[b]-thiophen der Formel

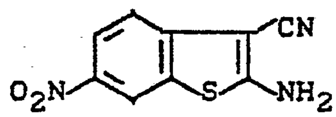

Zu einer Suspension von 5,53 g (25 mmol) p-Nitrophenylcyanthioacetamid und 4,2 g (50 mmol) Natriumhydrogencarbonat in 40 ml 1,2-Dichlorethan tropft man eine Lösung von 1,3 ml (25 mmol) Brom in 10 ml Dichlorethan zu. Durch Kühlung mit Eiswasser hält man die Temperatur bei 20°C. Man läßt 30 Minuten nachrühren, saugt ab und wäscht mit Wasser nach. Nach Trocknen im Vakuum bei 50°C verbleiben 4,9 g eines braunen Pulvers = 89% d. Th.

$m_e^+$: 219; Fp.: 266 °C

IR: 3390 $cm^{-1}$ ($NH_2$); 2217 $cm^{-1}$ (C≡N)

1328 $cm^{-1}$ ($NO_2$)

$^1$H-NMR (DMSO): δ = 7,35 ppm (d, I = 7,5 Hz, 1H), 8,06 ppm (dd, I = 7,5 Hz, I = 2,5 Hz, 1H); 8,46 ppm (s, 2H, mit $D_2O$ austauschbar); 8,65 ppm (d, I = 2,5 Hz, 1H)

b) Darstellung von p-Nitrophenyl-cyanthioacetamid

Eine Lösung von 187 g p-Nitrophenylmalodinitril und 75 g Thioacetamid in 1 l Dimethylformamid wird bei Raumtemperatur mit Chlorwasserstoff gesättigt und unter weiterem schwachem Einleiten von Chlorwasserstoff auf 100°C erwärmt. Man rührt 90 Minuten bei 100°C. Nach Abkühlen trägt man die Lösung auf 4 l Eiswasser aus und hält dabei durch Zugabe von konzentrierter Natronlauge den pH-Wert bei 10 bis 11. Man klärt von einem geringen Rückstand und säuert das Filtrat durch Zugabe von konzentrierter Salzsäure unter Kühlung bis pH = 2 an. Zur besseren Kristallisation läßt man über Nacht bei Raumtemperatur nachrühren, saugt ab und trocknet.

Ausbeute: 143 g (65% der Theorie);

$m_e^+$: 221 (11%) $C_9H_7N_3O_2S$;

Fp.: 75°C

c) Darstellung von p-Nitrophenylmalodinitril

Zu einer Lösung von 120 g NaOH pulverisiert in 1,5 l Dimethylformamid fügt man 198 g Malodinitril und tropft anschließend bei Raumtemperatur eine Lösung von 236 g 4-Nitrochlorbenzol in 150 ml DMF zu. Man rührt zwei Tage bei Raumtemperatur, zieht das Lösungsmittel im Vakuum weitgehend ab und löst den Rückstand in 5 l Eiswasser. Man stellt unter Kühlung mit Salzsäure auf pH = 2, saugt den gebildeten Niederschlag ab und trocknet im Vakuum bei 50°C.

Ausbeute: 240 g oranges Pulver (Rohausbeute. 85% der Theorie).

Beispiel 2

a) Darstellung von 2-Amino-3-cyan-4-chlor-6-nitrobenzo-[b]-thiophen der Formel

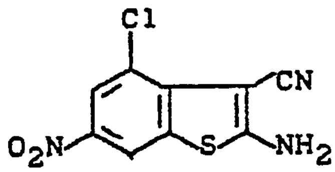

Zu einer Suspension von 46 g 2-Chlor-4-nitrophenylcyanthioacetamid (0,18 Mol) und 30 g Natriumhydrogencarbonat (0,36 Mol) in 650 ml Methylenchlorid tropfte man bei Raumtemperatur eine Lösung von 9,2 ml (0,18 Mol) Brom in 72 ml Methylenchlorid zu. Man rührte zwei Stunden bei Raumtemperatur, saugte ab und wusch den gelben Rückstand mehrmals mit Wasser.
Ausbeute: 43 g = 94% der Theorie
$m_e^+$: 253 (100%); 255 (33,8%) ($C_9H_4ClN_3O_2S$);
Fp.: 279°C

b) Darstellung von 2-Chlor-4-nitrophenyl-cyanthioacetamid

Eine Lösung von 66,5 g (0,3 Mol) 2-Chlor-4-nitrophenylmalodinitril und 22,5 g (0,3 Mol) Cyanthioacetamid in 300 ml Dimethylformamid wird bei Raumtemperatur mit Chlorwasserstoff gesättigt und unter weiterem Einleiten von Chlorwasserstoff auf 100°C erwärmt. Nach zwei Stunden bei 100°C läßt man abkühlen und trägt auf 1,5 l Eiswasser aus, wobei durch Zugabe von konzentrierter Natronlauge pH = 10,5 gehalten wird. Man klärt von einem unlöslichen Rückstand und säuert das Filtrat unter Eiskühlung langsam mit konzentrierter Salzsäure bis pH = 2 an. Die zunächst ölige Fällung kristallisiert über Nacht durch.
Ausbeute: 55 g = 72% der Theorie;
$m_e^+$: 255 ($C_9H_6ClN_3O_2S$);
Fp.: 148°C
$^1$H-NMR (DMSO) δ = 5,9 ppm (s, 1H mit $D_2O$ austauschbar); 7,85 ppm (d, 1H); 8,28 ppm (m, 2H); 9,75 ppm (s, 1H, mit $D_2O$ austauschbar); 10,23 ppm (s, 1H, mit $D_2O$ austauschbar)

c) Darstellung von 2-Chlor-4-nitrophenylmalodinitril

Zu einer Lösung von 40 g pulverisiertem Natriumhydroxid und 66 g (1 Mol) Malodinitril in 1 l Dimethylformamid tropfte man bei 0°C in einer Stunde eine Lösung von 96 g (0,5 Mol ) 1,2-Dichlor-4-nitrobenzol in 100 ml Dimethylformamid. Man läßt zwei Tage bei Raumtemperatur nachrühren, zieht das Lösungsmittel im Vakuum ab und trägt den Rückstand auf 4 l Eiswasser aus. Unter Kühlung stellt man die Lösung langsam auf pH = 1,5 durch Zutropfen von konzentrierter Salzsäure. Man saugt ab und wäscht mit 1n Salzsäure nach. Man trocknet im Vakuum bei 50°C.
Ausbeute: 105 g (95% der Theorie)
$m_e^+$: 221 (54%), 223 (18%);
$^1$H-NMR (DMSO): δ = 6,95 ppm (d, I = 9 Hz, 1H);
7,83 ppm (m, 2H); 12,5 ppm (s breit, 1H);
Fp.: 105°C

<u>Beispiel 3</u>

a) Darstellung von 2-Amino-3-cyan-4-trifluormethyl-6-nitro-benzo-[b]-thiophen

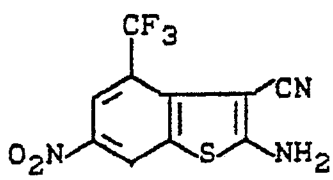

Man verfährt analog Beispiel 2a.
Ausbeute: 60% der Theorie;
$m_e^+$: 287 (199%) $C_{10}H_4F_3O_2S$;
Fp.: 257°C

b) Darstellung von 2-Trifluormethyl-4-nitrophenylcyanthioacetamid als Ausgangsmaterial für Beispiel 3a.

Eine Lösung von 230 g 2-Trifluormethyl-4-nitrophenyl-malodinitril in 900 ml Dimethylformamid wird bei Raumtemperatur mit Chlorwasserstoff gesättigt. Nach Zugabe von 74,4 g Thioacetamid wird 2 Stunden auf 100°C erwärmt. Man läßt auf Raumtemperatur abkühlen und gießt auf 6 l Eiswasser, wobei durch Zugabe von Natronlauge pH 10 bis 11 gehalten wird. Nach Klärfiltrat wird das Filtrat unter Kühlung langsam mit konzentrierter Salzsäure auf pH = 2 gestellt. Der semikristalline Niederschlag wird abgetrennt und über Nacht mit 2 l Wasser und 1 l Methanol verrührt. Man saugt ab und wäscht mit 1n Salzsäure.
Ausbeute: 191 g (65% der Theorie)
Fp.: 73°C
c) Darstellung von 2-Trifluormethyl-4-nitrophenylmalodinitril
Man verfährt analog Beispiel 1c und erzielt eine Ausbeute von 93% der Theorie;
Fp.: 103°C

Beispiel 4

Darstellung von 2-Amino-3-cyan-4-methylsulfonyl-6-nitrobenzo-[b]-thiophen der Formel

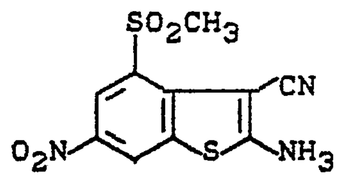

Die Synthese erfolgt analog Beispiel 1 aus 2-Methylsulfonyl-4-nitrophenyl-cyanthioacetamid. Das Rohprodukt wird aus Dimethylformamid umkristallisiert.
$m_e^+$: 297 (100%) $C_{10}H_7N_3O_4S_2$;
IR: 2200 $cm^{-1}$ (C ≡ N);
$^1$H-NMR (DMSO) δ = 3,30 ppm (s, 4H, -$NH_2$, $H_2O$); 3,45 ppm (s, 3H); 8,60 ppm (d, I = 2 Hz, 1H); 8,85 ppm (s, breit, 1H); 9,05 ppm (d, I = 2 Hz, 1H).

Beispiel 5

Darstellung von 2-Amino-3-cyan-6,7-dichlor-benzo-[b]-thiophen der Formel

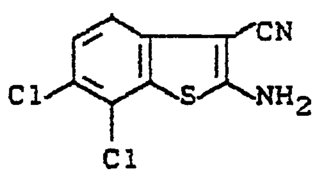

Durch Umsetzung von 3,4-Dichlorphenyl-malodinitril mit Thioacetamid analog Beispiel 1 wird 3,4-Dichlorphenylcyanthioacetamid in 63%iger Ausbeute erhalten. Fp.: 143°C (3,4-Dichlorphenylmalonitril läßt sich durch Reaktion von 3,4-Dichlorbenzylcyanid mit Dimethylcarbonat/Natriummethylat, weitere Umsetzung mit Chlorcyan, Verseifung und Decarboxylierung erhalten).
Der Ringschlug von 3,4-Dichlorphenyl-cyan-thioacetamid zum 2-Amino-3-cyan-6,7-di-chlor-benzo-[b]-thiophen erfolgt analog Beispiel 6 mit 69%iger Ausbeute. Das Produkt läßt sich aus Dimethylformamid umkristallisieren.
$m_e^+$: 242 (100%), 243 (12% ), 244 (76% ), 245 (7% ), 246 (7% ), 246 (14%), ($C_9H_4Cl_2N_2S$);
Fp.: 262°C;
$^1$H-NMR (DMSO) δ = 7,2 ppm (d, I = 7,5 Hz, 1H); 7,48 ppm (d, I = 7,5 Hz, 1H); 8,18 ppm (s, 2H, mit $D_2O$ austauschbar).

**Patentansprüche**

1. Verfahren zur Herstellung von s-Aminothiophenen der Formel

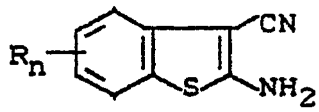

worin
R einen Substituenten und
n eine Zahl von 0 bis 4 bedeuten,
dadurch gekennzeichnet, daß man Thioamide der Formel

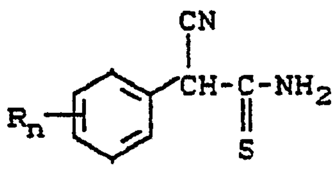

worin R und n die obengenannte Bedeutung haben, mit Dehydrierungsmitteln ringschließend umsetzt.

2. Verfahren nach Anspruch 1, dadurch gekennzeichnet, daß man Halogen oder halogenabspaltende Verbindungen als Dehydrierungsmittel verwendet.

3. Verfahren nach Anspruch 1, dadurch gekennzeichnet, daß man Brom als Dehydrierungsmittel verwendet.

4. Verfahren nach Anspruch 1, dadurch gekennzeichnet, daß man die Umsetzung in Gegenwart eines Säureakzeptors durchführt.

5. Verfahren zur Herstellung von Verbindungen der Formel

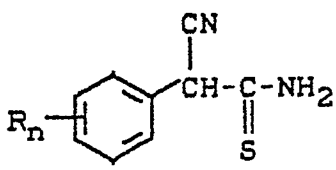

dadurch gekennzeichnet, daß man Verbindungen der Formel

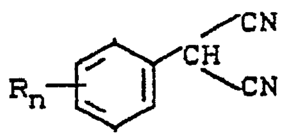

worin R und n die obengenannte Bedeutung haben, mit Verbindungen der Formel

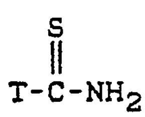

worin T $NH_2$, Aryl oder Alkyl bedeutet, in Gegenwart einer Säure umsetzt.

## Revendications

1. Procédé pour la fabrication de 2-aminothiophènes de formule

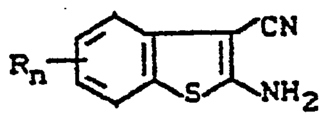

dans laquelle
R représente un substituant et
n représente est un entier de 0 à 4,
caractérisé en ce que l'on fait réagir des thioamides de formule

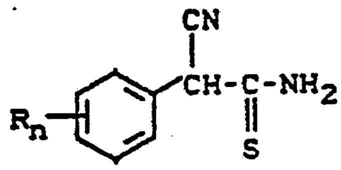

dans laquelle
R et n ont la signification ci-dessus, dans une réaction de cyclisation avec des agents déshydrogénants.

2. Procédé selon la revendication 1, caractérisé en ce que l'on utilise comme agents déshydrogénants un halogène ou des composés libérant un halogène.

3. Procédé selon la revendication 1, caractérisé en ce que l'on utilise le brome comme agent déshydrogénant.

4. Procédé selon la revendication 1, caractérisé en ce que l'on met en œuvre la réaction en présence d'un accepteur d'acide.

5. Procédé pour La fabrication de composés de formule

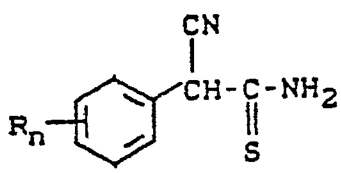

caractérisé en ce que l'on fait réagir des composés de formule

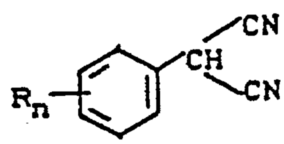

dans laquelle
R et n ont la signification ci-dessus avec des composés de formule

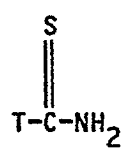

dans laquelle
T représente $NH_2$ ou un groupe aryle ou alkyle, en présence d'un acide.

## Claims

1. Process for preparing 2-aminothiophenes of the formula

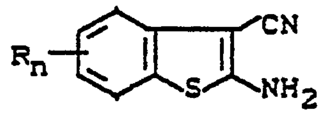

in which
R denotes a substituent and
n is a number from 0 to 4,
characterized in that thioamides of the formula

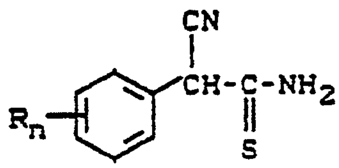

in which
R and n have the abovementioned meaning, are reacted with dehydrogenating agents to give ring closure.

2. Process according to Claim 1, characterized in that halogen or halogen-eliminating compounds are used as dehydrogenating agents.

3. Process according to Claim 1, characterized in that bromine is used as dehydrogenating agent.

4. Process according to Claim 1, characterized in that the reaction is carried out in the presence of an acid acceptor.

5. Process for preparing compounds of the formula

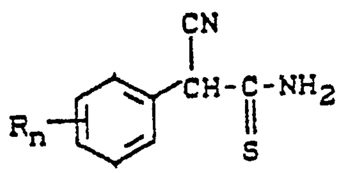

characterized in that compounds of the formula

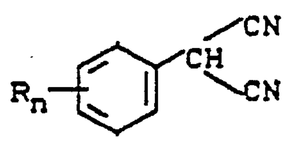

in which
R and n have the abovementioned meaning, are reacted with compounds of the formula

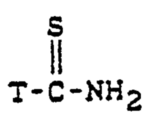

in which
T denotes $NH_2$, aryl or alkyl, in the presence of an acid.